(19) Europäisches Patentamt
European Patent Office
Office européen des brevets

(11) Veröffentlichungsnummer: **0 039 844**
**A2**

(12) # EUROPÄISCHE PATENTANMELDUNG

(21) Anmeldenummer: **81103265.5**

(51) Int. Cl.³: **C 07 D 311/62**

(22) Anmeldetag: **22.11.78**

(30) Priorität: **25.11.77 CH 14479/77**
**17.03.78 CH 2937/78**

(43) Veröffentlichungstag der Anmeldung:
**18.11.81 Patentblatt 81/46**

(84) Benannte Vertragsstaaten:
**BE CH DE FR GB LU NL SE**

(60) Veröffentlichungsnummer der früheren Anmeldung nach Art. 76 EPÜ: **0 003 274**

(71) Anmelder: **Zyma SA**
**Route de l'Etraz**
**CH-1260 Nyon(CH)**

(72) Erfinder: **Albert, Alban, Dr.**
**62, Av. de Gennecy**
**CH-1249 Avully(CH)**

(72) Erfinder: **Courbat, Pierre, Dr.**
**20, Route de Divonne**
**CH-1260 Nyon(CH)**

(72) Erfinder: **Weith, André, Dr.**
**24 B, Route du Curson**
**CH-1197 Prangins(CH)**

(74) Vertreter: **Meyer, Hans Rudolf et al,**
**Patentabteilung der CIBA-GEIGY AG Postfach**
**CH-4002 Basel(CH)**

(54) **Verfahren zur Herstellung von O-substituierten Derivaten des (+)-Cyanidan-3-ols.**

(57) Die Erfindung betrifft ein Verfahren zur Herstellung von O-substituierten Derivaten des (+)-Cyanidan-3-ols der Formel I

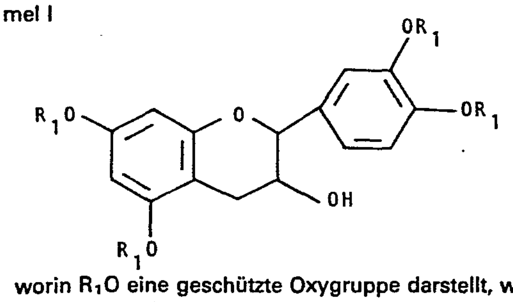

(1)

worin $R_1O$ eine geschützte Oxygruppe darstellt, wobei man ein Tetra-alkali-salz von (+)-Cyanidan-3-ol mit einem reaktionsfähigen Ester eines Alkohols der Formel $HO-R_1$ umsetzt. Dabei entstehen grösstenteils neue Verbindungen, die ebenfalls Gegenstand der Erfindung sind, und die als Ausgangsprodukte für die neuen Verbindungen gemäss der Europäischen Patentanmeldung 78810024.6 verwendet werden können.

EP 0 039 844 A2

Croydon Printing Company Ltd.

4-11475/1+2/+/B

# Verfahren zur Herstellung von O-substituierten Derivaten des (+)-Cyanidan-3-ols.

Die Erfindung betrifft ein Verfahren zur Herstellung von O-substituierten Derivaten des (+)-Cyanidan-3-ols, insbesondere solche der Formel I

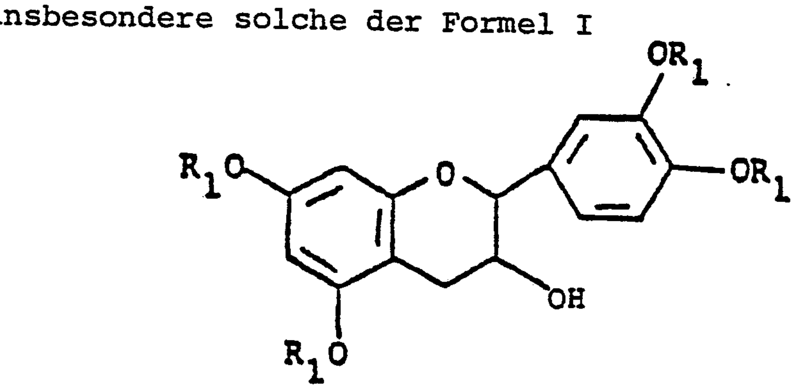

(I)

worin $R_1O$ eine geschützte Oxygruppe darstellt, die leicht hydrolisierbar oder hydrogenolysierbar ist.

In den Verbindungen der Formel I kann der Rest $R_1$ ein Niederalkylrest, der mono-, di- oder polysubstituiert ist, ein Niederalkenylrest, einen unsubstituierten oder mono-, di- oder trisubstituierten araliphatischen Rest, einen aliphatischen, aromatischen oder araliphatischen Carbonsäurerest oder ein veresterter Kohlensäurerest, oder ein Rest der mit dem an $R_1$ gebundenen Sauerstoffatom ein cyclisches Acetal bildet.

Wenn $R_1$ ein Niederalkylrest darstellt enthält dieser vorzugsweise 1-4 Kohlenstoffatome und ist in erster Linie Methyl oder Aethyl. Diese Reste können durch Niederalkoxy, z.B. Aethoxy oder Methoxy, oder Niederalkoxy-aethoxy, z.B. Methoxyäthoxy substituiert sein.

Ist $R_1$ ein Niederalkenylrest, enthält dieser vorzugsweise 2-5 Kohlenstoffatome und ist in erster Linie Allyl, Methallyl, 2-Butenyl oder 2-Pentenyl.

Ist $R_1$ ein araliphatischer, insbesondere ein Benzylrest, kann dieser verschieden substituiert sein, z.B. durch 1 oder mehrere Alkyl mit 1-4 Kohlenstoffatomen, wie Methyl, Aethyl, Isopropyl oder Tert.-Propyl, durch ein oder mehrere Halogene vorzugsweise Chlor, Brom oder Fluor, ein oder mehrere Alkoxy, wie Methoxy oder Aethoxy oder auch durch Nitril. Diese Substituenten können die ortho- und para-Stellung besetzen; bevorzugt sind sie in para-Stellung. Ist ein Substituent im Rest $R_1$ von zweiter Ordnung wie die Nitrogruppe, sitzt er vorzugsweise in meta-Stellung.

Ist der Rest $R_1$ ein aliphatischer Acylrest, ist der aliphatische Teil z.B. ein Alkyl mit 1-4 Kohlenstoffatomen und vorzugsweise Methyl oder Aethyl, die durch Niederalkoxy,

wie Methoxy substituiert sein können. Ist $R_1$ ein aromatischer oder araliphatischer Acylrest, ist der aromatische Rest besonders ein Phenylrest der gegebenenfalls substituiert ist, und der aliphatische Rest ein Alkyl mit 1-2 Kohlenstoffatomen.

Ist $R_1$ ein Alkoxycarbonyl, kann der Alkylrest wiederum durch ein Aryl substituiert sein oder durch ein Halogenatom. Ist $R_1$ ein Acetal zusammen mit dem Sauerstoff das den Rest $R_1$ trägt, kann dieses Acetal linear oder cyclisch sein, beispielsweise, ein Pyranylrest.

Unter den Resten der Formel $R_1$ sollen besonders genannt werden, z.B. Allyl, Methallyl, Aethylallyl, 2-Butenyl, 2-Pentenyl, Methoxymethyl, Aethoxymethyl, Methoxyäthoxymethyl, Acetonyl, Propionylmethyl, Cinnamyl, Phenacyl, Benzyl, 2-Methylbenzyl, 4-Methylbenzyl, 4-t-Butyl-benzyl, 4-Brombenzyl, 4-Fluorbenzyl, 4-Chlorbenzyl, 4-Methoxybenzyl, 4-Aethoxybenzyl, 3-Nitrobenzyl, 3,5-Dinitrobenzyl, 4-Cyanbenzyl, Chloro-, Bromo- oder Phenyl-phenacyl oder Methoxycarbonyl, Aethoxycarbonyl, 2,2,2-Trichloräthoxycarbonyl, oder Phenoxycarbonyl.

Die Verbindungen welche eine salzbildende Gruppe enthalten, wie beispielsweise Carboxylgruppen können in freier Form oder in Form ihrer Salze vorhanden sein. Diese Formen können gegenseitig Eine in die Andere übergeführt werden. Salze von Verbindungen die eine freie Carbonsäure enthalten, sind z.B. Metallsalze, im besondern Alkalimetallsalze z.B. Natrium oder Kaliumsalze, wie auch Erdalkalimetallsalze, z.B. Magnesium- oder Kalziumsalze, oder Ammoniumsalze z.B. mit Ammoniak oder organischen Basen, wie Triniederalkylamin, z.B. Trimethylamin oder Triäthylamin. Man erhält diese Salze z.B. indem man die freie Verbindung mit Hydroxyden oder Carbonaten dieser Metalle oder mit

Ammoniak oder Aminen, oder auch mit geeigneten Ionenaustauschern, oder mit metall-organischen Verbindungen umsetzt.

Verbindungen die basische Gruppen aufweisen, können sich auch in Form ihrer Säureadditionssalze, besonders mit nicht giftigen pharmazeutisch verwendbaren Säuren, z.B. mit Mineralsäuren, wie Salzsäure, Bromwasserstoffsäure, Schwefelsäure oder Phosphorsäure, oder mit organischen Carbon oder Sulfonsäuren z.B. aliphatischen, cycloaliphatischen, cycloaliphatischen-aliphatisch, aromatischen, araliphatischen, heterocyclischen oder heterocyclisch-aliphatischen Säuren, z.B. Essigsäure, Propionsäure, Bernsteinsäure, Glyconsäure, Milchsäure, Maleinsäure, Weinsäure, Zitronensäure, Ascorbinsäure, Phenylessigsäure, Benzoesäure, 4-Aminobenzoesäure, Anthranilsäure, 4-Hydroxybenzoesäure, Salicylsäure, Aminosalicylsäure, Embonsäure oder Nikotinsäure, wie auch Methansulfonsäure, Aethansulfonsäure, 2-Hydroxyäthansulfonsäure, Aethylensulfonsäure, Phenylsulfonsäure, p-Methylphenylsulfonsäure, Naphthalinsulfonsäure, Sulfanylsäure, oder Cyclohexylsulfaminsäure. Diese Salze können z.B. durch Behandlung der freien Verbindungen die eine basische Gruppe enthalten mit den genannten Säuren oder entsprechenden Anion-Austauschern behandelt werden.

Die Verbindungen der Formel I sind zum Teil bekannt, zum grössern Teil jedoch neu. Sie können durch eine neue und vorteilhafte Weise gewonnen werden, nämlich durch Reaktion des Tetraalkalimetallsalzes von (+)-Cyanidan-3-ol mit einem reaktionsfähigen Ester eines Alkohols der Formel

$$HO - R_1$$

worin $R_1$ die obengenannte Bedeutung hat.

Ein reaktionsfähiger Ester ist besonders ein Ester mit einer starken anorganischen Säure, in erster Linie mit einer Halogenwasserstoffsäure, wie Chlor- oder Bromwasserstoff oder einer organischen Sulfonsäure, vorzugsweise einer Niederalkansulfonsäure z.B. Methan- oder Aethansulfonsäure oder einer Benzolsulfonsäure die im Benzolkern z.B. durch Methyl, Chlor oder Brom substituiert sein kann, wie die p-Toluolsulfonsäure oder p-Brombenzolsulfonsäure.

Durch dieses Verfahren erhält man Derivate des (+)-cyanidan-3-ols deren vier phenolische Hydroxygruppen substituiert sind. Unter diesen Tetraäthern wurde das 5,7,3',4'-tetra-O-Benzyl-(+)-cyanidan-3-ol bereits durch ein reines Laboratoriumsverfahren hergestellt. [K. Weinges et D.Seiler, Liebigs Ann. Chem. 714 193-204 (1968)]. Dabei wurde (+)-Cyanidan-3-ol gelöst in Aceton mit Benzylchlorid in Gegenwart von Kaliumjodid und Kaliumcarbonat unter Stickstoff und mehrstündigem Kochen am Rückfluss umgesetzt. Es handelt sich dabei um eine klassische Benzylierung und sie führt nach Trennung und Kristallisationen aus Aethanol zu einer Mischung von 8-Benzyl-5,7,3',4'-tetra-O-Benzyl-(+)-cyanidan-3-ol und 5,7,3',4'-tetra-O-Benzyl-(+)-cyanidan-3-ol. Das Letztere wird durch Chromatographie mit einer Ausbeute von 1-2 % isoliert. Diese Ausbeute ist so klein, dass diese Methode zur Herstellung der tetrasubstituierten Verbindungen industriell nicht verwertet werden kann.

Es wurde gefunden, dass man diese Verbindungen mit sehr guter Ausbeute erhalten kann, wenn man (+)-Cyanidan-3-ol in einem aprotischen und organischen Lösungsmittel mit hoher Dielektrizitätskonstante mit einem Alkalihydrid oder Alkalicarbonat behandelt und das erhaltene Tetraalkalisalz mit einem reaktionsfähigen Ester der Formel

HO - $R_1$

umsetzt, wobei das Verhältnis Cyanidan-3-ol, Alkalihydrid und reaktionsfähiger Ester 1: ungefähr 4,25 : ungefähr 4,5 beträgt oder das Verhältnis Cyanidan-3-ol, Alkalicarbonat und reaktionsfähiger Ester 1: ungefähr 8 : ungefähr 6 beträgt.

Das Alkalihydrid ist besonders Natriumhydrid und wird vorzugsweise in Form einer Dispersion in Oel verwendet. Das Alkalicarbonat ist besonders Kaliumcarbonat.

Die verwendeten aprotischen Lösungsmittel sind besonders Amide, wie Dimethylformamid, aber auch Sulfoxyde, wie Dimethylsulfoxyde. Das Dimethylformamid wird jedoch aus ökonomischen Gründen vorgezogen, weil man es durch einfache Destillation bei Normaldruck wieder erhalten kann, und weil es einfacher zu handhaben ist als Dimethylsulfoxyd.

Im übrigen ist es wichtig, dass das Reaktionsmilieu so trocken als nur möglich ist. So trocknet man das Dimethylformamid über einem Molekularsieb und trocknet auf gleiche Weise die Lösung von Cyanidanol in Dimethylformamid. Die Bildung von Nebenprodukten wird dabei vermieden, ohne dass das Trocknen der Bildung der gewünschten Produkte entgegenstehe.

Die Reaktion kann in Gegenwart eines quaternären Ammoniumsalzes wie Tetra-n-butylammoniumhydrogensulfat, das in einer Proportion von 0,1 bis 0,5 Mol per 1 Mol Cyanidan-ol verwendet werden, oder eines Crown-äthers, vorzugsweise 18-Crown-6 der in einer Menge von 0,5 Mol per 1 Mol Cyanidan-ol verwendet wird, durchgeführt werden.

Man führt die Reaktion bei einer Temperatur zwischen -25°C und ungefähr +50°C durch. Da es bekannt ist, dass das Dimethylformamid sich bereits bei Raumtemperatur

im Kontakt mit basischen Substanzen zu zersetzen beginnt, führt man diese Reaktion vorzugsweise bei -25°C bis +25°C und in erster Linie bei ungefähr -5°C bis ungefähr 0°C durch. Mit Dimethylsulfoxyd, da es sich kaum zersetzt, kann man die Reaktion bei einer Temperatur die niederer ist als 50°C, vorzugsweise zwischen +15°C und ungefähr +30°C z.B. bei Raumtemperatur durchführen.

Da das Alkalisalz des (+)-Cyanidan-3-ols in Dimethylformamid-natriumhydrid selbst bei niederer Temperatur nicht stabil ist, muss man das Veresterungs- oder Verätherungsmittel sofort zugeben. Wird das nicht gemacht, wird die Reaktionsausbeute vermindert und die Reinigungsphase des gewünschten Produktes erschwert. Nach der Zugabe des Veresterungs- bzw. Verätherungsmittels bei tiefer Temperatur wird diese noch eine gewisse Zeit beibehalten bevor man langsam die Temperatur ansteigen lässt, z.B. auf Raumtemperatur. Die Reaktion ist dann beendet und die Reaktionsmischung kann mehr als 15 Stunden in diesem Zustand aufbewahrt werden ohne dass sie die Qualität des gewünschten Produktes verändert. Während der Reaktion ist gutes Rühren notwendig.

Das Fortschreiten der Reaktion kann durch Dünnschichtchromatographie auf Silikagel unter Verwendung von Chloroform oder Dichlormethan als mobile Phase, kontrolliert werden.

Nachdem das Lösungsmittel aus dem Reaktionsmedium durch Destillieren entfernt wurde, nimmt man den Rückstand in einem aprotischen Lösungsmittel, wie es zur Kristallisation verwendet wird, auf. Als Lösungsmittel zur Kristallisation des gewünschten Produktes eignet sich besonders Trichloräthylen. Auch andere Lösungsmittel wie Tetrachlorkohlenstoff, Toluol, Aethylacetat, Aethanol, Isopropanol oder Mischungen dieser Lösungsmittel z.B. mit Tetrachlorkohlenstoff,

n-Hexan, oder Aceton-methanol, geben gute Resultate. Aethylenäther und n-Hexan wie auch Aceton, Pyridin, Chloroform, Dimethylformamid, Dimethylsulfoxid, Tetrahydrofuran und Dichlormethan eignen sich weniger gut da die neuen Substanzen darin bei Raumtemperatur löslich sind.

Die nachfolgenden Beispiele illustrieren die oben beschriebene Erfindung; sie sollen jedoch diese in ihrem Umfang in keiner Weise einschränken. Temperaturen werden in Celsius-Graden angegeben.

Die erfindungsgemäss erhältlichen Verbindungen können als Zwischenprodukte, z.B. zur Herstellung von O-substituierten Derivaten des (+)-cyanidan-3-ols verwendet werden, wie sie in der Europäischen Patentanmeldung 78810024.6 beschrieben sind.

Beispiel 1

In einem 50 ml Kolben mit einem Zufüllstutzen, einer Stickstoffzuführung und einem Kühler, der ein Calziumchloridrohr trägt, gibt man 0,96 g einer 55 %igen Dispersion von Natriumhydrid in Oel und trocknet das Ganze durch Erwärmen im Stickstoffstrom. Nach dem Abkühlen unter einem schwachen Stickstoffstrom gibt man 10 ml frisch destilliertes Dimethylsulfoxyd zu und rührt bei Raumtemperatur. Man fügt tropfenweise eine Lösung von 1,45 g (+)-Cyanidan-3-ol in 15 ml frisch destilliertem Dimethylsulfoxyd zu wobei sich Wasserstoff entwickelt. 30 Minuten später gibt man eine Lösung von 2,74 ml Methoxyäthoxymethylchlorid in 10 ml Dimethylsulfoxyd zu und giesst 30 Minuten später die Reaktionslösung in 150 ml mit Kochsalz gesättigtem Wasser, und stellt

pH auf 7. Man erhält ein klebriger braunschwarzer Niederschlag den man 3 Mal mit 100 ml Toluol extrahiert. Die erhaltenen Lösungen werden vereinigt, und 3 Mal mit 50 ml Wasser gewaschen. Man entfernt das Toluol und extrahiert das erhaltene braunrote Oel 10 Mal mit 100 ml n-Hexan am Rückfluss. Die n-Hexan-Lösungen ergeben das 5,7,3',4'-tetra-O-Methoxyäthoxymethyl-(+)-cyanidan-3-ol in Form eines gelben Oels, das bei 150°C/0,02 mmHg zersetzt wird.

Beispiel 2

Unter Stickstoff und gutem Rühren stellt man eine Suspension von 92,8 g einer 55 %igen Dispersion von Natriumhydrid in Oel (51 g NaH) in einem Liter frisch destilliertem Dimethylformamyd her. Man kühlt sie auf eine Temperatur zwischen -5 und 0°C ab und gibt dazu eine Lösung von 145 g (+)-Cyanidan-3-ol in 2 Liter frisch destilliertem Dimethylformamyd zu, so dass die Dispersionstemperatur konstant bleibt. Entstehender Wasserstoff wird durch zirkulierenden Stickstoff entfernt.

Nach der Einführung vom (+)-Cyanidan-3-ol lässt man bei tiefer Temperatur während 15 Minuten stehen, gibt dann im Laufe von 1 1/2 Stunden 267,5 ml Benzylbromid zu, so dass die Temperatur nicht über 0°C steigt. Man lässt bei dieser Temperatur nochmals eine halbe Stunde stehen und dann auf Raumtemperatur erwärmt.

Man destilliert im Vakuum bei 60°C das Dimethylformamid ab, nimmt den öligen Rückstand in 3 Liter Trichloräthylen auf, wäscht die organische Lösung mit 3 Liter destilliertem Wasser und filtriert. Man engt ein und kühlt ab und erhält so das 5,7,3',4'-tetra-O-Benzyl-(+)-cyanidan-3-ol das in weissen Nadeln kristallisiert. F=144-145°C.

Beispiel 3

Unter den gleichen Bedingungen wie in Beispiel 2, jedoch unter Verwendung von Dimethylformamid das sorgfältig getrocknet worden ist, erhält man das 5,7,3',4'-tetra-O-Benzyl-(+)-cyanidan-3-ol.

Beispiel 4

Analog Beispiel 2, jedoch unter Verwendung von 2,25 Mol Benzylchlorid, erhält man das Tetra-O-benzyl-5,7,3',4'-(+)-cyanidan-3-ol.

Beispiel 5

Zu einer Lösung von 0,93 g Natriumhydriddispersion in Oel (0,51 g NaH) in 10 ml Dimethylsulfoxyd das frisch destilliert wurde, gibt man langsam unter Rühren und bei Raumtemperatur gibt man eine Lösung von 1,45 g (+)-Cyanidan-3-ol in 20 ml frisch destilliertem Dimethylsulfoxyd. Der entstehende Wasserstoff wird durch zirkulierenden Stickstoff entfernt. Nach einer Stunde Rühren bei Raumtemperatur hört die Stickstoffentwicklung praktisch auf und das Natriumsalz des (+)-Cyanidan-3-ols ist zum Teil an der Kolbenwand abgeschieden. Man gibt dann tropfenweise 2,68 ml Benzylbromid zu. Man lässt 1 1/2 Stunden unter Rühren bei Raumtemperatur stehen, gibt die Reaktionslösung langsam und unter Rühren in 300 ml einer kalten wässrigen, 10 %igen Lösung von Kochsalz. Der Niederschlag wird abfiltriert, und im Vakuum bei 80°C getrocknet, dann aus 40 ml Tetrachlorkohlstoff umstristallisiert. Man erhält 1,96 g 5,7,3',4'-tetra-O-Benzyl-(+)-cyanidan-3-ol.

Beispiel 6

Man verfährt wie in Beispiel 2, verwendet jedoch 416 g α-Brom-o-xylen anstelle von Benzylbromid. Der Rückstand der nach dem Abdampfen des Dimethylformamids erhalten wird, wird in Chloroform aufgenommen, die Lösung mit destilliertem Wasser gewaschen, filtriert und eingedampft. Man kristallisiert den Rückstand aus einer Mischung von Benzol und Petroläther (6:3 v/v). Man erhält so das 5,7,3',4'-tetra-O-(2-methyl-benzyl)-(+)-cyanidan-3-ol vom F 88-90°C.

Beispiel 7

Analog Beispiel 2, jedoch unter Verwendung von 416 g α-Brom-p-xylol anstelle von Benzylbromid erhält man das 5,7,3',4'-tetra-O-(4-methyl-benzyl)-(+)-cyanidan-3-ol das nach Umkristallisieren aus Tetrachlorkohlenstoff bei 66-70°C schmilzt.

Beispiel 8

Analog Beispiel 2, aber unter Verwendung von 562 g 2-Brombenzylbromid anstelle von Benzylbromid erhält man das 5,7,3',4'-tetra-O-(2-Brom-benzyl)-(+)-cyanidan-3-ol, das aus einer Mischung von Chloroform und Tetrachlorkohlenstoff (40:55 v/v) umkristallisiert wird. F=155-157°C.

Beispiel 9

Analog Beispiel 2, jedoch unter Verwendung von 181 g Chlordimethyläther anstelle von Benzylbromid erhält man das 5,7,3',4'-tetra-O-Methoxymethyl-(+)-cyanidan-3-ol, das nach Umkristallisieren aus einer Mischung von Wasser mit Methanol (1:1 v/v) bei 92-93°C schmilzt.

Beispiel 10

Analog Beispiel 2, jedoch unter Verwendung von 272 g Allylbromid anstelle Benzylbromid erhält man das 5,7,3',4'-tetra-O-Allyl-(+)-cyanidan-3-ol das nach dem Umkristallisieren aus n-Hexan bei 78,5-80°C schmilzt.

Beispiel 11

Analog Beispiel 37, jedoch unter Verwendung von 244 g Aethylchloroformiate anstelle von Benzylbromid erhält man das 5,7,3',4'-tetra-O-Aethoxycarbonyl-(+)-cyanidan-3-ol dessen Schmelzpunkt ungefähr bei 65°C liegt.

Beispiel 12

Man löst 1,45 g (+)-cyanidan-3-ol in 50 ml wasserfreiem Aceton, gibt 6,91 g wasserfreies Kaliumcarbonat zu und erhitzt ihn am Rückfluss im Stickstoff. Zu dieser Lösung gibt man eine Lösung von 6,26 g p-Brom-phenacyl-bromid in 30 ml wasserfreiem Aceton und lässt weiter kochen unter mechanischem Rühren während 3 Stunden. Nach dem Abkühlen der Reaktionsmischung filtriert man die Salze ab und dampft die Lösung zur Trockne ein. Den Rückstand nimmt man in 50 ml Chloroform auf, wäscht die Chloroformlösung 3 Mal mit 50 ml Wasser, dampft das Chloroform ab, löst den Rückstand in ungefähr 1000 ml Aether, filtriert und engt die Aetherlösung auf 150 ml ein. Man erhält so das 5,7,3',4'-tetra-O-(4-Brom-phenacyl)-(+)-cyanidan-3-ol, das nach dem Umkristallisieren auf Aethanol bei 127-129°C schmilzt.

Beispiel 13

Man gibt in einen 1 Liter Kolben 110 g Kaliumcarbonat unter schwachem Stickstoff, dann 250 ml Dimethylformamid, und giesst eine Lösung von 29,0 g (+)-Cyanidan-3-ol in 250 ml getrocknetem Dimethylformamid und 71,25 ml Benzylbromid gelöst in 100 ml Dimethylformamid. Man erwärmt auf 100°C während 5 1/2 Stunden unter Rühren und im Stickstoffstrom. Die erhaltene Suspension wird heiss filtriert, das Filtrat bei 80°C im Vakuum am Rotavapor eingedampft und der Rückstand mit 250 ml Chloroform gelöst. Die Chloroformlösung wird filtriert, 3 Mal mit 250 ml Wasser gewaschen, über Magnesiumsulfat getrocknet und zur Trockne eingedampft. Man nimmt dem Rückstand in 750 ml am Rückfluss kochenden Tetrachlorkohlenstoff auf, trennt den braunen klebrigen Rückstand ab und lässt bei Raumtemperatur unter Rühren und gelegentlichem Abkühlen im Eisschrank kristallieren. Das erhaltene Produkt ist identisch mit dem des Beispiels 2 F=144-145°C.

Beispiel 14

Ein 500 ml Kolben wird mit einem mechanischen Rührer, einen Zuflussstutzer, einem Kalziumchloridrohr und einer Zuführung für Stickstoff ausgerüstet. Das Ganze wird getrocknet und und unter Stickstoff gesetzt bevor man 100 ml wasserfreies Dimethylformamid zugibt. Man kühlt auf -3°C, gibt 9,28 g einer öligen Dispersion von Natriumhydrid (55 %) und dann unter starkem Rühren eine Lösung von 14,5 g (+)-Cyanidan-3-ol in 200 ml wasserfreiem Dimethylformamid, 1,698 g Tetra-n-butylammoniumhydrogensulfat zu und hält unter Rühren 1 Stunde bei 0°C. Man gibt dann innerhalb 15 Minuten eine

Lösung von 25,9 ml Benzylchlorid in 25 ml wasserfreiem Dimethylformamid zu und rührt während 30 Minuten bei 0°C. Man lässt die Temperatur auf Raumtemperatur steigen, hält das Reaktionsgemisch während 24 Stunden unter starkem Rühren, filtriert und dampft unter vermindertem Druck ein. Der Rückstand wird in 250 ml Chloroform aufgenommen, die Lösung 4 Mal mit 250 ml destilliertem Wasser gewaschen, über Magnesiumsulfat getrocknet, filtriert und eingedampft. Der Rückstand wird mit 50 ml Hexan gewaschen und dann auf Gewichtskonstanz gebracht. Diese Masse wird heiss in 500 ml Tetrachlorkohlenstoff gelöst, heiss filtriert und durch Abkühlen auf Raumtemperatur und anschliessendem Einengen auf 350 ml und 150 ml das 5,7,3',4'-tetra-O-Benzyl-(+)-cyanidan-3-ol das mit dem im Beispiel 37 erhaltenen Produkt identisch ist.

Beispiel 15

Wie in Beispiel 13 gibt man nach der Einführung der Cyanidanollösung 13,22 g 18-Crown-6 und ersetzt das Benzylbromid durch 69 ml Benzylchlorid. Man erwärmt dann während 20 Stunden unter starkem Rühren auf 60°C. Man erhält so das 5,7,3',4'-tetra-O-Benzyl-(+)-cyanidan-3-ol das mit dem im Beispiel 2 erhaltenen Produkt identisch ist.

Beispiel 16

Man verfährt analog Beispiel 15, ersetzt jedoch das 18-Crown-6 durch 16,98 g Tetra-n-butylammoniumhydrogensulfat. Man erhält 44,5 g 5,7,3',4'-tetra-O-Benzyl-(+)-cyanidan-3-ol.

Patentansprüche:

1. Verfahren zur Herstellung von O-substituierten Derivaten des (+)-Cyanidan-3-ols der Formel I

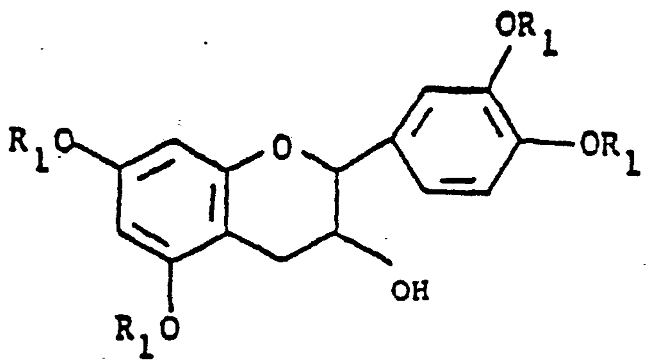

(I)

worin $R_1O$ eine geschützte Oxygruppe darstellt, dadurch gekennzeichnet, dass man ein Tetra-alkali-salz von (+)-Cyanidan-3-ol mit einem reaktionsfähigen Ester eines Alkohols der Formel $HO-R_1$ umsetzt.

2. Verfahren gemäss Anspruch 1, dadurch gekennzeichnet, dass man (+) Cyanidan-3-ol in einem aprotischen und organischen Lösungsmittel mit hoher Dielektrizitätskonstante, mit einem Alkalihydrid behandelt und das erhaltene Tetraalkalisalz mit einem reaktionsfähigen Ester der Formel HOR umsetzt, wobei das Verhältnis Cyanidan-3-ol, Alkalihydrid und reaktionsfähiger Ester 1: ungefähr 4,25: ungefähr 4,5 beträgt.

3. Verfahren gemäss Anspruch 1, dadurch gekennzeichnet, dass man (+) Cyanidan-3-ol in einem aprotischen und organischen Lösungsmittel mit hoher Dielektrizitätskonstante, mit einem Alkalicarbonat behandelt und das erhaltene Tetraalkalisalz mit einem reaktionsfähigen Ester der Formel

HOR umsetzt, wobei das Verhältnis Cyanidan-3-ol, Alkalicarbonat und reaktionsfähiger Ester 1: ungefähr 8: ungefähr 6 beträgt.

4. O-substituierte Derivate des (+)-Cyanidan-3-ols der Formel Ia

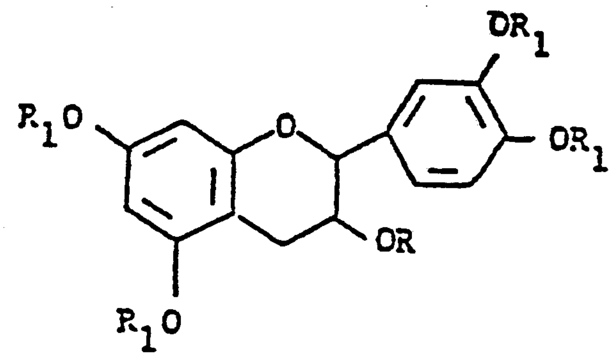

(Ia)

worin OR die im Anspruch 1 gegebene Bedeutung oder Wasserstoff ist und $OR_1$ eine geschützte Hydroxgruppe darstellt, die leicht hydrier- oder hydrolisierbar ist, mit der Ausnahme derjenigen Verbindungen, worin alle Reste $R_1$ Benzyl und R Wasserstoff oder Methyl ist.